# EUROPÄISCHE PATENTSCHRIFT

(11) **EP 2 223 707 B1**
(45) Veröffentlichungstag und Bekanntmachung des Hinweises auf die Patenterteilung: **09.04.2014**
(21) Anmeldenummer: 09153728.2
(22) Anmeldetag: 26.02.2009
(51) Int. Cl.: A61L 27/00, A61L 24/04, A61F 2/28

(54) **Implantatsystem und Knochenimplantat**
Implant system and bone implant
Système d'implant et implant osseux

(43) Veröffentlichungstag der Anmeldung: 01.09.2010
(73) Patentinhaber: New Dent AG, 4702 Oensingen (CH)
(72) Erfinder: Perler, Peter, 2562 Port (CH); Schwenter, Peter, 2545 Selzach (CH); Staudenmann, Roger, 3292 Busswil bei Büren (CH)
(74) Vertreter: Raffay & Fleck

(56) Entgegenhaltungen:
- WO-A-95/28124
- US-A- 5 990 194
- US-A1- 2002 177 102
- US-A1- 2003 083 662

## Beschreibung

Die Erfindung betrifft ein Implantatsystem mit einem Knochenimplantat und einem Verankerungsmittel zum Verankern des Knochenimplantates in einem Knochen. Sie betrifft ferner ein Knochenimplantat zur Verwendung in einem solchen Implantatsystem.

Es ist in der Medizintechnik bekannt, Implantate zur festen und dauerhaften Verbindung mit Knochen bereitzustellen, die entweder selbst bereits z.B. als Ersatz für verschlissene Körperteile dienende Funktionsteile enthalten oder auf die prothetisch weiter aufgebaut werden kann. So werden derartige Knochenimplantate beispielsweise beim Bilden künstlicher Gelenke zum Ersetzen krankhaft verschlissener natürlicher Gelenke verwendet (z.B. Knochenprothesen am Oberschenkelknochen zum Ausbilden eines künstlichen Femurkopfes). Auch sind Knochenprothesen im Dentalbereich bekannt, die im Kiefer verankert werden und als Basis zum Fixieren eines künstlichen Zahnersatzes dienen.

Dabei ist allgemein bekannt, dass für eine dauerhaft feste und spielfreie Verbindung des Knochenimplantates mit dem umgebenden Knochen, also für eine bestmögliche Osseointegration, die Erlangung einer hohen Anfangsfestigkeit und Stabilität der Anbindung des Knochenimplantates an den umgebenden Knochen von höchster Wichtigkeit ist. Entsprechend werden hier in hohem Maße Anstrengungen unternommen, um einen festen und stabilen Erstsitz eines Knochenimplantates zu erreichen.

Dabei gibt es neben einfachen rein mechanischen Lösungen in Form von in Presspassung eingesetzten und mit hoher Kraft in den Knochen eingedrückten Implantaten bzw. Schraubimplantaten Lösungen, die mit einem zusätzlichen Verankerungsmittel arbeiten. Bekannt ist herbei insbesondere die Anwendung von sogenanntem Knochenzement. Bei Knochenzement handelt es sich um eine viskose Mischung aus Monomeren oder kurzen Polymeren und ggf. einem Polymerisator, wobei diese Mischung bei der Implantation in den Bereich der Verbindung zwischen Knochenimplantat und Knochen gegeben wird und dort in situ polymerisiert, also längerkettige Polymere ausbildet und so erstarrt. Bei diesem Erstarrungsvorgang ergibt sich dann ein Formschluss einerseits zwischen dem Knochenimplantat und dem Polymer, andererseits zwischen dem Knochen, insbesondere dem Knocheninneren, der sogenannten Substantia Spongiosa und dem Polymer, wodurch letztlich der feste Halt des Implantates im Knochen hervorgerufen wird.

Eine weitere bekannte Maßnahme besteht darin, gemeinsam mit dem Knochenimplantat ein Quantum an festem, thermoplastischem Polymer in die Implantationsstelle einzubringen und dieses Polymer nach dem Einsetzen des Implantates zu erwärmen und zu verflüssigen. Dies geschieht beispielsweise mittels hochfrequenter mechanischer Schwingungen, insbesondere Ultraschall. Denkbar sind aber auch andere Möglichkeiten der Erhitzung, z.B. Laser oder dgl. Das so erhitzte thermoplastische Polymer verflüssigt sich und fließt in die porösen Zwischenräume des Knochenmaterials im Bereich der Substantia Spongiosa, kühlt dort ab und erstarrt, so dass eine feste Verbindung geschaffen wird.

Beispiele für die oben genannten Verankerungsmethoden befinden sich hinsichtlich des oben beschriebenen Knochenzementes in der DE 22 29 702, hinsichtlich der Methode der temporären Verflüssigung des thermoplastischen Polymers z.B. in der WO 2005/079696.

In der US 2002/0177102 A1 ist ein Dentalimplantat in Form einer in den Kiefer einzubringenden Schraube gezeigt. Diese weist einen quer zu ihrer Längserstreckung verlaufenden Kanal auf mit Austrittsmündungen. In einer anderen Variante ist ein Längskanal gezeigt, der gerade geführt ist bis zu einer Austrittsmündung am Ende des Kanals. Durch diese Kanäle kann eine Nadel einer Spritze eingeführt werden, durch die ein biokompatibles, aushärtbares Material (z.B. Knochenzement oder ein polymerisierendes Material) in den Kieferknochen in die Umgebung des Implantats eingebracht wird.

Auch wenn sich mit den genannten Methoden, insbesondere denjenigen, die ein zusätzliches Verankerungsmittel einsetzen, hohe Primärstabilitäten erreichen lassen, die übermäßige Relativbewegungen zwischen Knochenimplantat und Knochen ausschließen, jedenfalls solche oberhalb der in der Literatur diskutierten Obergrenzen von zwischen 0,02 - 0,05 mm, weisen diese auch Nachteile auf. So sind insbesondere mit einer Schraubverbindung eingesetzte Knochenimplantatkörper in ihrem in dem Knochen sitzenden Halteabschnitt zwangsläufig rotationssymmetrisch ausgebildet, was naturgemäß Probleme bei der Verdrehsicherheit bzw. Verdrehstabilität hervorruft. Auch ist es bei diesen Implantaten nicht möglich, ein einmal gesetztes Implantat in der Position zu korrigieren, ohne einen Verlust an Primärstabilität hinnehmen zu müssen. Ein geschraubtes Knochenimplantat kann in derselben vorbereiteten Implantationsstruktur im Knochen (typischerweise ein zylinderförmiges Loch) nicht noch einmal mit korrigierter Position eingesetzt werden, ohne an Halt zu verlieren. Schließlich ist die für geschraubte Knochenimplantate zwingend vorgegebene rotationssymmetrische Ausbildung des Halteabschnittes insbesondere dort auch von Nachteil, wo die Knochenstruktur z.B. besonders schmal ist und die Anbringung entsprechend rotationssymmetrischer Löcher zum Einsetzen des Implantates nicht bzw. nicht ohne einen erheblichen Verlust von Knochensubstanz gestattet.

Die Verwendung von Knochenzement bringt aufgrund der stets exothermen Polymerisationsreaktion einen hohen Wärmeeintrag in das die Implantationsstelle umgebende Gewebe mit sich. Da die Polymerisation des Knochenzements in einer vergleichsweise kurzen Zeit abläuft, ergibt sich zudem ein hoher Energieeintrag in vergleichsweise kurzer Zeit, was zu thermisch bedingten Schädigungen des umgebenden Gewebes führen kann. Darüber hinaus ist das Knochenzement auch nicht biokompatibel abbaubar im Körper und verbleibt so als Langzeitimplantat.

Bei der weiteren oben aufgezeigten Möglichkeit, ein Knochenimplantat über temporär aufgeschmolzene thermoplastische Polymere im Knochen zu verankern, ist zunächst die vergleichsweise kostspielige und vorzuhaltende Implantationsausrüstung erforderlich, z.B. ein Gerät beinhaltend einen Ultraschallgenerator, einen Piezowandler und eine Sonotrode zum Verflüssigen des thermoplastischen Materials mit Ultraschall. Ferner ist auch hier von Nachteil, dass ein Wärmeeintrag in das die Implantationsstelle umgebende Gewebe erfolgt und nicht verhindert werden kann.

Hier soll nun mit der Erfindung Abhilfe geschaffen werden, indem ein Implantatsystem angegeben wird, welches eine vergleichsweise zuverlässige Verankerung im Knochen bei hoher Primärstabilität erlaubt, ohne die Nachteile der aus dem Stand der Technik bekannten Verfahren mit sich zu bringen. In einem weiteren Aspekt der Erfindung soll ein neuartiges Knochenimplantat bereitgestellt werden, welches sich zur Verwendung in einem erfindungsgemäßen Implantatsystem eignet.

Gelöst wird diese Aufgabe in ihren unterschiedlichen Aspekten durch zunächst ein Kieferknochenimplantat gemäß dem Anspruch 1, zu dem vorteilhafte Weiterbildungen in den abhängigen Ansprüchen 2 bis 5 angegeben sind; ferner durch ein Implantatsystem mit den Merkmalen des Anspruches 6 sowie den diesbezüglichen vorteilhaften Weiterbildungen gemäß den abhängigen Ansprüchen 7 bis 13.

Erfindungsgemäß hat das Knochenimplantat einen in seinem Inneren verlaufenden Kanal, der einerseits eine Zugangsöffnung außerhalb des Halteabschnittes aufweist, von der aus er zugänglich ist und der andererseits in wenigstens einer Austrittsöffnung in einem Bereich des Halteabschnittes mündet. Durch diesen Kanal kann von der Zugangsöffnung her bei der Anwendung des Knochenimplantates ein Verankerungsmittel in Form eines durch Lösen verflüssigten Polymers eingebracht, z.B. mit einer Injektionsspritze eingespritzt werden. Aufgrund seiner Viskosität fließt das gelöste Polymer durch den Kanal und tritt durch die Austrittsöffnung aus und in das umgebende Knochengewebe, insbesondere die Substantia Spongiosa ein. Dabei verbleibt allerdings nach wie vor zumindest ein Rest des verflüssigten Polymers im Inneren des Kanals, wo dieser Rest durch Kontakt mit Körperflüssigkeit und das sich so ergebende Ausspülen des Lösungsmittels ebenfalls erstarrt. Damit aber wird eine feste und sichere Verbindung zwischen dem Knochenmaterial und dem Knochenimplantat über das mit beiden genannten Elementen in Verbindung stehende und fest verankerte Polymer - nach dessen Erstarren - geschaffen. Erfindungswesentlich ist auch, dass, Um ein besonders sicheres Ausströmen des verflüssigten Polymers und Eindringen desselben in das umgebende Knochenmaterial zu erleichtern, im Bereich der Austrittsöffnung bzw. der Austrittsöffnungen der Kanal oder ein mit dem Kanal verbundener Stichkanal in Richtung des distalen, also zuerst in den Knochen eindringenden Endes des Halteabschnittes geneigt ist. Dabei können die entsprechenden Austrittsöffnungen mit unterschiedlich geneigtem Verlauf des Kanals bzw. der dort mündenden Stichkanäle gebildet sein, z.B. ein steilerer, mehr in Richtung einer Längsachse des Implantates geneigter Verlauf in einer dem distalen Ende ferneren Position und ein flacherer, mehr in Richtung einer senkrechten zu der Längsachse geneigter Verlauf in Richtung des distalen Endes.

Das Knochenimplantat besteht zumindest in dem Halteabschnitt bevorzugt aus einem biokompatiblen Metall, einer biokompatiblen Keramik oder einem biokompatiblen Kunststoff oder einer beliebigen Kombination dieser Materialien.

Um für eine besonders gute Verankerung des Halteabschnittes im umgebenden Knochenmaterial zu sorgen, weist das Knochenimplantat entlang des Umfanges des Halteabschnittes bevorzugt in unterschiedlichen Positionen angeordnet mehrere Austrittsöffnungen auf. Dabei können zusätzlich (ggf. auch alternativ) auch mehrere Austrittsöffnungen in axialer Richtung des Knochenimplantates, genauer des Halteabschnittes übereinander angeordnet sein.

Um eine noch bessere Verankerung des Polymers mit dem Körper des Knochenimplantates sicherzustellen, können im Bereich der Austrittsöffnung bzw. Austrittsöffnungen Hinterschnitte oder vergleichbare Strukturen zum gezielten Zurückhalten eines Teils des durch den Kanal und die Austrittsöffnung bzw. Austrittsöffnungen gepressten Verankerungsmittels vorgesehen sein. Solche Strukturen unterstützen noch einmal zusätzlich die Ausbildung einer festen Verbindung zwischen dem Polymer und dem Knochenimplantat beim Erstarren des Polymers.

Die Austrittsöffnung bzw. Austrittsöffnungen können als einfache Bohrungen oder vergleichbare vollständig freiliegende Öffnung ausgebildet sein, alternativ ist es aber auch möglich, diese Öffnungen als offenporöse Struktur in dem Halteabschnitt auszubilden. Eine solche offenporöse Struktur verbessert noch einmal zusätzlich die Verbindung zwischen dem Knochenimplantat und dem Polymer nach dessen Erhärten.

Der vorliegenden Erfindung liegt insbesondere hinsichtlich des Implantatsystems die Erkenntnis zugrunde, dass ein wie oben beschriebenes Knochenimplantat mittels eines unter Zugabe eines biokompatiblen und wasserlöslichen Lösungsmittels temporär verflüssigten Polymers im Knochen sicher verankert werden kann. Hierzu wird ein Polymer in der Vorbereitung und Bereitstellung durch Zugabe eines entsprechenden Quantums eines Lösungsmittels verflüssigt, indem die intermolekularen Verbindungen aufgehoben werden. Durch seine Wasserlöslichkeit wird das biokompatible Lösungsmittel an der Implantationsstelle durch die dort immer vorhandene, überwiegend aus Wasser bestehenden Körperflüssigkeiten aus der Mischung ausgespült, so dass das verflüssigte Polymer bzw. die Polymermischung wieder erhärtet.

Die bei diesem Vorgang frei werdende Erstarrungswärme ist bedeutend niedriger als z.B. die Wärmemenge, die bei der exothermen Reaktion des Knochenzements auftritt, so dass die diesbezüglichen schädlichen Einflüsse auf das umliegende Gewebe vermieden werden können. Auch ist es möglich, ein biologisch resorbierbares Polymer zu wählen, so dass nach erfolgter Osseointegration die mit diesem Verankerungsmittel gebildete Verankerungsstruktur aufgelöst wird und somit ein geringerer Anteil von Fremdkörpern an der Implantationsstelle verbleibt. In der Anwendung ist es für einen Implantologen insbesondere möglich, aufgrund einer während der Vorbereitung der Implantation vorgenommenen Einschätzung der Knochenqualität das Verankerungsmittel, also die Polymer-LösungsmittelMischung in seiner Menge entsprechend der Knochenqualität zu wählen. Verfügt der Knochen über eine ausgeprägte und gesunde Substantia Spongiosa, so benötigt er eine geringere Menge des Verankerungsmittels als in einem Knochen, dessen Substantia Spongiosa weniger gut ausgebildet oder durch Desintegration bereits zurückgebildet ist.

Grundsätzlich ist es im Rahmen der Erfindung auch möglich, ein Implantatsystem bereitzuhalten, das das Verankerungsmittel in noch ungelöstem Zustand, d.h. getrennt als zu verflüssigendes Polymer oder Polymergemisch und als wasserlösliches Lösungsmittel beinhaltet. Allerdings wird bevorzugt, das Verankerungsmittel bereits als fertige Mischung aus dem Polymer bzw. Polymergemisch und dem Lösungsmittel vorzuhalten, da auf diese Weise ein optimales Verhältnis zwischen Polymer und Lösungsmittel sichergestellt ist, das einerseits eine ausreichende Viskosität für die Applikation erlaubt, andererseits aber auch für ein möglichst schnelles Aushärten und Erstarren des Polymers am Implantationsort sorgt.

Für den Fall, dass ein bioresorbierbares Polymer in dem Verankerungsmittel Verwendung findet, sollte dieses vorzugsweise eine Abbauzeit im tierischen oder menschlichen Körper von 12 bis 36 Monaten aufweisen. Diese Abbauzeiten korrespondieren sehr gut mit einer ausreichenden Dauer einer Osseointegration, so dass am Ende des Abbaus des Polymers eine ausreichende Festigkeit im Knochen durch Einwachsung bzw. Osseointegration gegeben ist.

Als Polymer oder Polymergemisch kommen insbesondere die in Anspruch 5 genannten Substanzen und Materialien in Betracht, wobei derzeit die Gruppe der Polylactide besonders bevorzugt wird. Das Lösungsmittel wird bevorzugt aus einem der in Anspruch 6 angegebenen Stoffe bzw. einer Kombination oder Mischung derselben gewählt, wobei hier das derzeit bevorzugte Lösungsmittel ein N-Methyl-2-Pyrrolidon ist.

Um die Osseointegration zu befördern, kann das flüssige Verankerungsmittel neben dem wasserlöslichen biokompatiblen Lösungsmittel und dem Polymer oder Polymergemisch ferner ein Knochenersatzmaterial enthalten, welches insbesondere Tricalciumphosphat und/oder Hydroxiapatit sein kann. Ein solches Knochenersatzmaterial wirkt sich insbesondere für die Knochenbildung positiv aus, wenn es in Form von porösen Teilchen, insbesondere Kügelchen, beigemengt ist, wobei die Größen dieser Teilchen zwischen 0,02 und 0,7 mm liegen sollten.

Das erfindungsgemäße Implantatsystem eignet sich zum Verankern unterschiedlichst gebildeter Implantate, es kann beispielsweise auch für geschraubte Knochenimplantate verwendet werden. Für solche bildet es an der Implantationsstelle um den in den Knochen eingeschraubten Halteabschnitt des Knochenimplantates herum einen mit dem Knochen verbundenen "Anker" bzw. eine Art Dübel, der bei einem bioresorbierbaren Polymer als Bestandteil des Verankerungsmittels mit der Zeit abgebaut und durch echte Knochensubstanz ersetzt werden kann.

Besonders effektiv lässt sich das erfindungsgemäße Implantatsystem aber mit einem Knochenimplantat verwenden, wie es in Anspruch 9 gekennzeichnet ist. Demnach weist das Knochenimplantat einen Halteabschnitt und einen mit diesem verbundenen Funktionsabschnitt auf. Der Halteabschnitt ist typischerweise der Abschnitt, der in den Knochen an der Implantationsstelle angebracht wird, der Funktionsabschnitt hingegen übernimmt die eigentliche Funktion des Implantates, beispielsweise in Form eines Abschnittes eines künstlichen Gelenkes oder aber als Basis für ein darauf aufzusetzendes, getrenntes Implantat, beispielsweise eine Zahnprothese oder dgl.

Zum Festlegen eines Knochenimplantates unter Zuhilfenahme des erfindungsgemäßen Systems wird an der Implantationsstelle zunächst eine entsprechende Aufnahmeöffnung für das Implantat in dem Knochen vorbereitet. Dies geschieht in üblicher Weise, z.B. durch Bohren.

Dann wird zunächst dieses Implantat passgerecht in die im Knochen vorbereitete Öffnung eingesetzt und ausgerichtet. Sodann wird durch Applikation des verflüssigten Polymers als Verankerungsmittel in die Zugangsöffnung zu dem Kanal dieses verflüssige Polymer durch den Kanal hindurch und aus der Austrittsöffnung bzw. den Austrittsöffnungen im Halteabschnitt des Knochenimplantates heraus gedrückt und in die Knochenstruktur, insbesondere die Substantia Spongiosa eingepresst. Durch den Kontakt mit der im Bereich der Implantationsstelle stets vorhandenen Körperflüssigkeit wird das wasserlösliche Lösungsmittel nach und nach ausgewaschen, so dass das Polymer bzw. das Polymergemisch erstarrt und erhärtet unter Ausbildung einer festen Verbindung einerseits zu dem Knochen, andererseits zu dem Knochenimplantat, wo eine ausreichende Menge des Polymers im Inneren des Kanals verbleibt und einstückig mit dem in dem Knochenmaterial erstarrten Polymer in Verbindung steht.

Wenn, wie bevorzugt, für das Polymer ein bioresorbierbarer Kunststoff, z.B. Polylactid, verwendet wird, so wird dieses Material über einen längeren Zeitraum abgebaut und durch Knochenmasse ersetzt, so dass am Ende nur noch das Knochenimplantat verbleibt in vollständig osseointegrierter Form.

Weitere Vorteile und Merkmale der Erfindung ergeben sich aus der nachfolgenden Beschreibung eines Ausführungsbeispiels anhand der beigefügten Figuren. Dabei zeigen:
- Fig. 1: in einer Seitenansicht ein erstes Ausführungsbeispiel eines erfindungsgemäßen Knochenimplantates zur Verwendung in einem erfindungsgemäßen Implantatsystem;
- Fig. 2: das erfindungsgemäße Knochenimplantat aus Fig. 1 gesehen entlang einer Schnittlinie genommen entlang der in Fig. 1 gezeigten Schnittlinie A-A;
- Fig. 3: in schematischer Darstellung und teilweise weggeschnitten ein Knochenimplantat eingesetzt in einem hier schematisch dargestellten Kieferknochen;
- Fig. 4: in einer dreidimensionalen schematischen Ansicht ein zweites Ausführungsbeispiel für ein erfindungsgemäßes Knochenimplantat zur Verwendung in einem erfindungsgemäßen Implantatsystem, speziell für den Einsatz in schmal gebildeten Knochen; und
- Fig. 5: in einer schematischen Darstellung ein wie in Fig. 4 gezeigtes Knochenimplantat eingesetzt in einem schematisch dargestellten Kieferknochen.

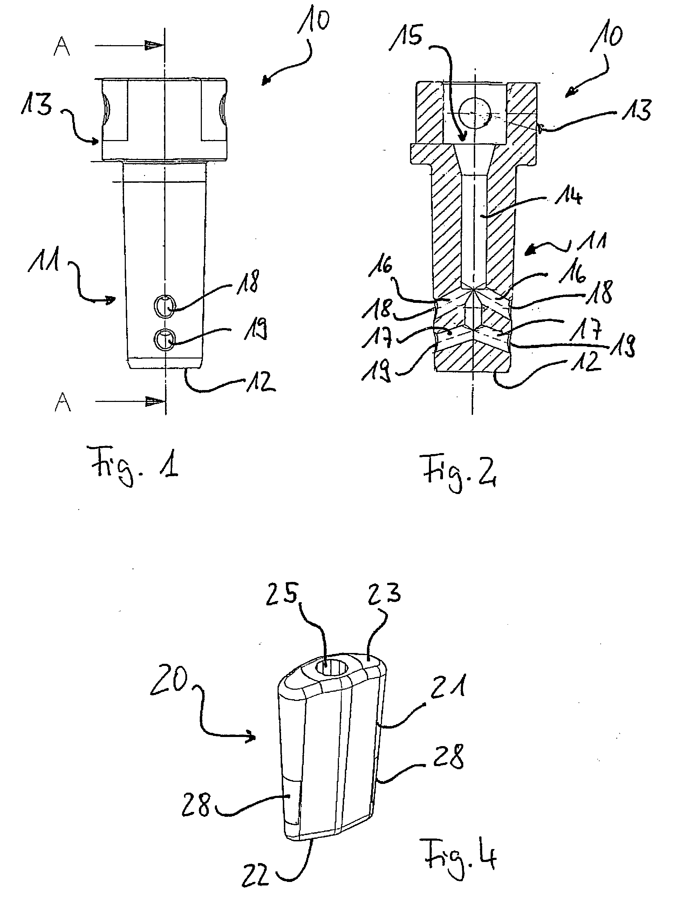

In den Figuren 1 bis 5 sind in unterschiedlichen Darstellungen zwei Ausführungsbeispiele eines erfindungsgemäßen Knochenimplantates gezeigt, teilweise eingesetzt in einen schematisch dargestellten Knochen.

Anhand der Figuren werden nachstehend nun nicht nur die beiden Ausführungsbeispiele für erfindungsgemäße Knochenimplantate, sondern auch entsprechende Ausführungsbeispiele eines erfindungsgemäßen Implantatsystems sowie das Verfahren zum Verankern der entsprechenden Knochenimplantate in dem Knochen am Implantationsort erläutert und beispielhaft beschrieben.

In den Figuren 1 und 2 ist in zwei unterschiedlichen Darstellungen, in einer Ansicht von der Seite sowie in einer Schnittdarstellung, ein erstes Ausführungsbeispiel für ein erfindungsgemäßes Knochenimplantat 10 gezeigt. Das hier gezeigte Knochenimplantat 10 ist ein für den Einsatz als Kieferimplantat im Mundbereich gestaltetes Implantat. Es setzt sich grob zusammen aus einem zum Einsetzen in den Knochen vorgesehenen Halteabschnitt 11 mit einem distalen Ende 12 und einem Funktionsabschnitt 13, der hier der Anbindung einer Zahnprothese dient. Das Knochenimplantat 10 ist einstückig gebildet, z.B. aus einem biokompatiblen Metall, wie etwa Titan der Qualität Grade4. Jedenfalls der Halteabschnitt 11 ist rotationssymmetrisch zu der Längsachse des Knochenimplantates 10 ausgebildet, wobei der Halteabschnitt 11 leicht konisch sich verjüngend zum distalen Ende 12 hin gearbeitet ist, was ein Einsetzen in den Knochen vereinfacht.

Im Inneren des Knochenimplantates 10 (vgl. Fig. 2) ist ein Kanal 14 ausgebildet, der im Wesentlichen entlang der Längsachse des Knochenimplantates 10 verläuft. An seiner dem Funktionsabschnitt 13 zugewandten Seite mündet der Kanal 14 in eine Zugangsöffnung 15, in Richtung des distalen Endes 12 des Knochenimplantates 10 zweigen diametral einander gegenüberliegend insgesamt vier, pro Seite je zwei Stichkanäle 16 bzw. 17 ab, die in Austrittsöffnungen 18 bzw. 19 münden. Dabei verlaufen die dem distalen Ende 12 zugewandten Stichkanäle 17 flacher als die vom distalen Ende 12 entfernten Stichkanäle 16, die steiler verlaufen.

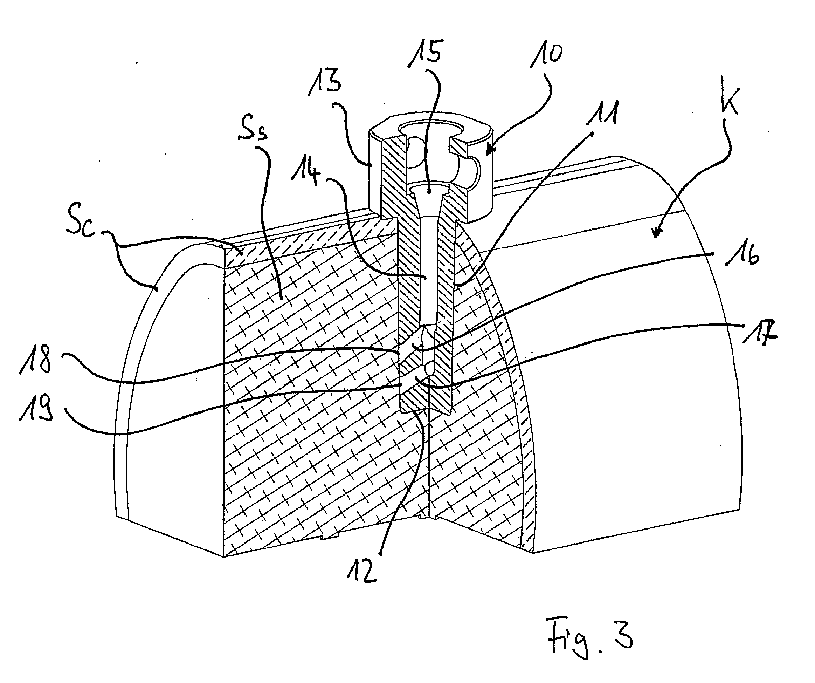

Wie in Fig. 1 zu erkennen ist, liegen die Austrittsöffnungen 18 und 19 der Stichkanäle 16 und 17 jeweils einer Seite entlang der Längsachse des Knochenimplantates 10 übereinander bzw. hintereinander angeordnet. In Fig. 3 ist in einer schematischen dreidimensionalen Ansicht, die teilweise weggeschnitten ist, das Knochenimplantat 10 eingesetzt in einen Knochen K, hier einen schematisch darstellten Kieferknochen, gezeigt. Das Knochenimplantat 10 sitzt mit seinem Halteabschnitt 11 in einem zuvor in üblicher Weise in den Knochen eingebrachten, beispielsweise gebohrten, Sackloch und ist dort in Passung eingesetzt. Dabei durchdringt der Halteabschnitt 11 die oberste, harte Knochenschicht, die Substantia Corticalis und ragt bis in den schwammartig aufgebauten porösen Bereich des Knochens, die Substantia Spongiosa hinein. Insbesondere liegen auch die Austrittsöffnungen 18 und 19 am Ende der Stichkanäle 16 und 17 im Bereich der Substantia Spongiosa, so dass dieser Abschnitt des Knochens K mit dem Kanal 14 in Verbindung steht.

Um das Knochenimplantat 10 nun zum Erreichen einer hohen Primärstabilität im Knochen K zu fixieren wird eine gemäß der Erfindung zusammen mit dem Knochenimplantat 10 ein erfindungsgemäßes Implantatsystem bildendes Verankerungsmittel verwendet. Dieses Verankerungsmittel besteht aus einem hier nicht näher dargestellten, mit einem wasserlöslichen, biokompatiblen Lösungsmittel gelösten Polymer, insbesondere einem gelösten Polylactid. Dieses kann über die konisch aufgeweitete Zugangsöffnung 15 z.B. mit einer mit entsprechendem konischen Anschluss versehenen Injektionsspritze in den Kanal 14 eingebracht und über die Stichkanäle 16 und 17 aus den Austrittsöffnungen 18 und 19 herausgepresst werden. Das aus den Austrittsöffnungen 18 und 19 austretende gelöste Polymer dringt in die porösen Zwischenräume zwischen der Knochensubstanz in der Substantia Spongiosa ein und hält dabei einen durchgehenden Verbund. Dieser Verbund ist darüber hinaus mit einem in den Stichkanälen 16 und 17 und in dem Kanal 15 verbleibenden Rest durchgehend verbunden. Nach dem Einbringen des mittels des Lösungsmittels verflüssigten Polymers wird das Lösungsmittel aufgrund des Kontaktes mit dem im Bereich der Implantationsstelle stets vorhandenen Körperflüssigkeiten und des in diesen Körperflüssigkeiten vorhandenen Wassers ausgewaschen, so dass das Polymer aus dem gelösten Zustand in einem festen Zustand übergeht, indem es eine stabile Verbindung mit dem Knochen K einerseits, genauer der Substantia Spongiosa dieses Knochens K, und dem Knochenimplantat 10 andererseits eingeht und letztgenanntes so im Knochen K fixiert.

Bevorzugt ist das gelöste Polymer bioresorbierbar, insbesondere ein Polylactid, so dass über einen durch die Wahl des Polymers steuerbaren Zeitraum, der idealer Weise 12 bis 36 Monate betragen sollte, das Polymer vollständig resorbiert und abgebaut wird. Das Polymer ist dann als Anker auch nicht mehr erforderlich, da das Implantat zwischenzeitlich durch Osseointegration fest mit dem Knochen verbunden ist. Um die Knochenbildung an der Implantationsstelle zusätzlich zu befördern, kann dem gelösten Polymer ein Knochenersatzmaterial, z.B. Tricalciumphosphat oder Hydroxiapatit, beigemengt sein, dies vorteilhafter Weise in Form von kleinen porösen Kügelchen mit Größen zwischen 0,02 und 0,7 mm.

Die oben beschriebene Verankerung des Knochenimplantates 10 in dem Knochen K kann einfach vorgenommen werden und ist sehr fest, was im Anschluss an die Beschreibung des zweiten Ausführungsbeispiels näher erläutert werden wird. Bei dieser Methode tritt keine lokale starke Erwärmung des die Implantationsstelle umgebenden Gewebes auf, und das Knochenimplantat 10 kann vor der Injektion des mit dem Lösungsmittel verflüssigten Polymers beliebig aus dem vorbereiteten Implantationsloch entnommen und wieder eingesetzt werden, um eine optimale Positionierung zu erreichen.

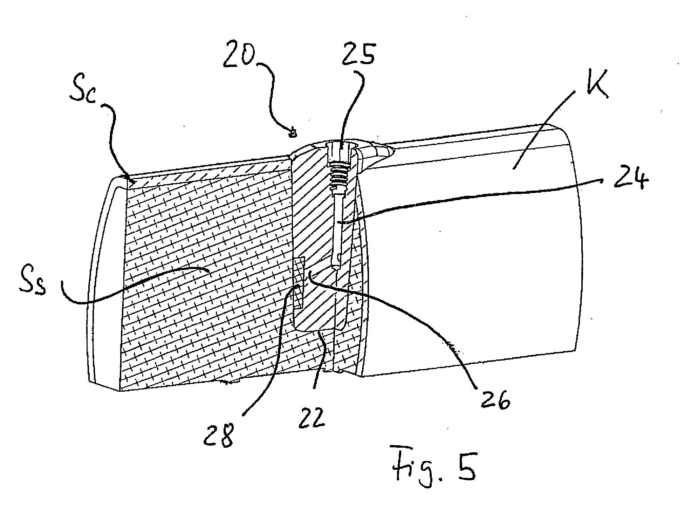

Eine zweite Ausgestaltungsform, die ebenfalls ein Kieferknochenimplantat zeigt und insbesondere für schmale Knochenbereich geeignet ist, ist in Fig. 4 in einer dreidimensionalen Ansicht und in Fig. 5 in einer teilweise weggeschnittenen Darstellung positioniert im Knochen K gezeigt.

Das hier gezeigte Ausführungsbeispiel eines Knochenimplantates 20 ist mit einem schmalen und keilförmig gebildeten Halteabschnitt 21 versehen, an den sich ein sehr flach ausgebildeter Funktionsabschnitt 23 mit in einer Zugangsöffnung 25 zu dem Kanal 24 (vgl. Fig. 5) ausgebildeten Haltestrukturen für eine Zahnprothese anschließt. Anders als im Fall des zuvor beschriebenen Knochenimplantates 10 ist das hier gezeigte Knochenimplantat 20 nicht etwa mit einem rotationssymmetrischen Halteabschnitt 21 versehen, sondern wie bereits erwähnt mit einem flachen keilförmigen solchen Abschnitt. Diese Ausgestaltung ermöglicht einerseits eine Anordnung des Knochenimplantates 20 in schmalen Knochenbereichen, es verhindert ferner die Gefahr eines Verdrehens des in den Knochen eingesetzten Knochenimplantates 20. Nahe dem distalen Ende 22 des Halteabschnittes 21 sind hier zwei langgestreckte Austrittsöffnungen 28 angeordnet, die wie in Fig. 5 zu erkennen ist, keine durchgängigen Öffnungen sind, sondern durch eine poröse Struktur in der Außenwandung des Knochenimplantates 20 in diesem Bereich gebildet sind. Zu diesen porösen Strukturen bzw. Austrittsöffnungen 28 führen Stichkanäle 26, die von dem Kanal 24 abzweigen.

Die Fixierung auch dieses Knochenimplantates 20 wird in analoger und gleicher Weise wie die Fixierung des Knochenimplantates 10 im Knochen K vorgenommen. Dies ist in Fig. 5 gezeigt, wobei dort der Knochen K bewusst schmaler gezeichnet ist, als im Falle des Knochenimplantates 10, um die Eignung des Knochenimplantates 20 zum Anbringen in schmalen bzw. filigranen Knochenstrukturen zu verdeutlichen.

Nachdem in üblicher Weise am Implantationsort ein den Außenabmessungen des Halteabschnittes 21 des Knochenimplantates 20 entsprechendes Sackloch in den Knochen K eingebracht worden ist, wird das Knochenimplantat 20 mit seinem Halteabschnitt 21 und dem distalen Ende 22 voran in dieses Sackloch eingesetzt und richtig positioniert. Ist die richtige Position des Knochenimplantates 20 gefunden, so kann die Primärfixierung erfolgen. Hierzu wird analog wie in dem zuvor beschriebenen Beispiel ein mit einem biokompatiblen, wasserlöslichen Lösungsmittel gelöstes Polymer in viskoser Form durch die Zugangsöffnung 25 in den Kanal 24 gedrückt, z.B. mit einer Injektionsspritze, und weiter durch die Stichkanäle 26 und die in Form von porösen Bereichen gebildeten Austrittsöffnungen 28 bis in die Substantia Spongiosa Ss des umgebenden Knochengewebes. In analoger Weise wie oben beschrieben wird das Lösungsmittel durch den Kontakt mit den Körperflüssigkeiten ausgespült, so dass das Polymer erstarrt und die Verankerung geschaffen ist. Die Ausbildung der Austrittsöffnung 28 als poröser Abschnitt in der Wandung des Implantates verbessert hier noch einmal die Verbindung zwischen dem erstarrten Polymer und dem Knochenimplantat 20 durch vergrößerte Oberflächenkontakte.

Aus der voranstehenden Beschreibung dürfte klar deutlich geworden sein, welche Vorteile die Verwendung erfindungsgemäßer Knochenimplantate bzw. erfindungsgemäßer Implantatsysteme mit sich bringen.

Nachfolgend wird noch hinsichtlich des Verankerungsmittels ein Ausführungsbeispiel sowie ein mit einem solchen durchgeführter Versuch beschrieben:

Zu Herstellung des Verankerungsmittels in Form eines gelösten Polymers wurde in einem Versuch das Polylactid LR706 des Herstellers Böhringer Ingelheim in Granulat-Form verwendet. Das Resomer LR706 ist ein Gemisch aus L-Lactid und R-Polymer. Durch Ringöffnungspolymerisation entstehen Polymere von L-Lactid und D-Lactid. Dabei weist LR706 ein Gemisch von 70:30 L-Lactid zu D-Lactid auf. Das Resomer LR706 ist amorph. Dies hat gegenüber reinem Poly-L-Lactid den Vorteil, dass letztgenannte teilkristallin sind und sich im Körper deutlich langsamer abbauen.

Als biokompatibles, wasserlösliches Lösungsmittel wurde ein N-Methyl-2-Pyrrolidon verwendet, das von dem Hersteller Sigma-Aldrich bezogen wurde unter dem Handelsnamen SIGMA-ALDRICH 691 17 1-Methyl-2- pyrrolidone purum; absolute.

Zur Herstellung der Polymer-Lösungsmittel-Masse wurden 0,666 g Resomer RL706 in 10g N-Methyl-2-Pyrrolidon gelöst. Die Lösung des LR706 kann bei Raumtemperatur beschleunigt werden, wenn der Mischbehälter in einem Wasserbad (Temperatur 20°C) mit Ultraschall (Frequenz 20 kHz) Agitation gestellt wird. Die Dauer bis zur vollständigen Lösung betrug bei den Versuchen 30 Minuten.

Diese Mischung wurde verwendet, um in vergleichenden Versuchen mit dem Knochenersatzmaterial "Open Cell Rigid Foam" des Herstellers Sawbones Messungen zur Bestimmung der Auszugskraft vorzunehmen. Dabei wurden wie in Fig. 1 gezeigte Implantatkörper aus Titan Grade4 mit einem Außendurchmesser von 4 mm am Halteabschnitt und 6 mm am Funktionsabschnitt, deren Länge über alles 14 mm betrug, verwendet. Die Länge des Halteabschnittes betrug hier 10 mm, wobei die unteren 9 mm konisch verjüngt waren. Zehn dieser Versuchsimplantate wurden in jeweils ein Implantatbett in dem Knochenersatzmaterial eingesetzt, wobei als Implantatbetten mit einem Metallbohrer insgesamt zehn, für jeden der Implantatkörper eines, zylindrische Löcher mit einem Durchmesser von 3,6 mm und einer Tiefe von 10 mm gebohrt worden. Durch die konische Ausgestaltung des Halteabschnittes des Implantatkörpers entsteht eine Presspassung zwischen Implantatkörper und umgebenden Knochenersatzmaterial, bei dem es sich um einen offenporigen Urethanschaum mit der Dichte 0,12 pro Kubikzentimeter und einer durchschnittlichen Porengröße von 1,5 - 2,5 mm handelt.

Nachdem in jedes der Implantatbetten je ein Versuchsimplantat gedrückt worden war, wurde der Polyurethanschaumblock in einem Becken mit Ringerlösung (Bichsel, Interlaken REF 100 0 104) über fünf Minuten eingetaucht. Der Flüssigkeitsspiegel stand dabei etwa 1 mm über dem Schaumblock. Dies diente der Simulation der Durchdringung eines Knochens mit Körperflüssigkeit.

Mit einer 2,5 ml Spritze mit Kanüle rosa (1,2 x 30 mm) wurde in fünf der Implantatkörper je ein Kubikzentimeter der Polymer-Lösungsmittel-Masse gedrückt. Anschließend blieb der Polyurethanschaumblock während weiterer 10 Minuten in der Ringerlösung. Um die Durchblutung in den Knochen zu simulieren wurde die Ringerlösung mit einer Pumpe zusätzlich umgewälzt.

Anschließend wurden die Implantatkörper mit einer Geschwindigkeit von 0,01 m pro Sekunde aus dem Polyurethanschaumblock gezogen, und es wurde die Zugkraft aufgezeichnet und der Spitzenwert gespeichert.

Bei diesem Auszugtest ergab sich, dass die Implantate ohne eingebrachte Polymer-Lösemittel-Masse im Durchschnitt eine Auszugskraft von 3 N zeigten, während die Implantatkörper mit der Polymer-Lösungsmittel-Masse durchschnittlich eine um den Faktor 5 höhere Auszugskraft von 15 N zeigten. Das Versagen bei den letztgenannten Implantatkörpern trat innerhalb des Polyurethanschaumblockes, der den Knochen simuliert, auf und nicht etwa zwischen dem Implantatkörper und der ausgehärteten Polymer-Lösungsmittel-Masse. Insoweit wird erwartet, dass die Auszugskraft in einem Knochen mit einer äußeren harten Schicht aus Substantia Corticalis bzw. Substantia Compacta deutlich höher liegen wird.

Um zu verhindern, dass nach einem Einsetzen eines Implantates, wie beispielsweise in Fig. 1 gezeigt, in einen Kieferknochen und dem Aushärten des verwendeten Polymers und insbesondere dessen Resorption ein offener Kanal von der Mundhöhle oder einem anderen umgebenden Bereich in den Knochen entsteht, sollte die zentrale Bohrung im Inneren des Knochenimplantates 10 bzw. 20 verschlossen werden. Dies kann beispielsweise mittels eines Stopfens geschehen, welcher in den Kanal gerückt wird. Erfolgt eine solche Maßnahme nicht, besteht die Gefahr, dass durch einen solchen offenen Kanal Bakterien aus der Umgebung in den Knochen gelangen und dort Entzündungen hervorrufen können.

Auch die beschriebenen Versuche haben gezeigt, welchen Vorteil das erfindungsgemäße Implantatsystem in Verbindung mit dem verbindungsgemäßen Knochenimplantat mit sich bringt.

### Bezugszeichenliste

- 10: Knochenimplantat
- 11: Halteabschnitt
- 12: distales Ende
- 13: Funktionsabschnitt
- 14: Kanal
- 15: Zugangsöffnung
- 16: Stichkanal
- 17: Stichkanal
- 18: Austrittsöffnung
- 19: Austrittsöffnung
- 20: Knochenimplantat
- 21: Halteabschnitt
- 22: distales Ende
- 23: Funktionsabschnitt
- 24: Kanal
- 25: Zugangsöffnung
- 26: Stichkanal
- 28: Austrittsöffnung

- K: Knochen
- Sc: Substantia corticalis
- Ss: Substantia spongiosa

## Patentansprüche

1. Kieferknochenimplantat zum Einsetzen in einen menschlichen oder tierischen Kieferknochen (K) eines lebenden Körpers für den dauerhaften Verbleib und zur Verwendung mit einem mittels eines biokompatiblen, wasserlöslichen Lösungsmittels gelösten und so verflüssigten Polymers oder Polymergemischs als Verankerungsmittel zum Verankern des Knochenimplantates in einem Knochen, wobei das Knochenimplantat einen in den Knochen (K) einzusetzenden Halteabschnitt (11, 21) und einen mit dem Halteabschnitt (11, 21) verbundenen Funktionsabschnitt (13, 23) sowie wenigstens einen von einer Zugangsöffnung (15, 25) außerhalb des Halteabschnittes (11, 21) her zugänglichen, im Innern des Knochenimplantates (10, 20) verlaufenden Kanal (14, 24) aufweist, der in einem Bereich des Halteabschnittes (11, 21) in wenigstens einer Austrittsöffnung (18, 19, 28) mündet, **dadurch gekennzeichnet, dass** im Bereich der wenigstens einen Austrittsöffnung (18, 19, 28) der Kanal (14) oder ein mit dem Kanal (14) verbundener Stichkanal (16, 17, 26) in Richtung eines distalen Endes (12, 22) des Halteabschnittes (11, 21) geneigt verläuft.

2. Kieferknochenimplantat nach Anspruch 1, **dadurch gekennzeichnet, dass** es zumindest in dem Haltabschnitt (11, 21) aus einem biokompatiblen Metall, einer biokompatiblen Keramik oder einem biokompatiblen Kunststoff oder einer beliebigen Kombination dieser Materialien besteht.

3. Kieferknochenimplantat nach einem der vorhergehenden Ansprüche, **dadurch gekennzeichnet, dass** es mehrere Austrittsöffnungen (18, 19, 28) aufweist, die an unterschiedlichen Positionen des Umfanges des Halteabschnittes (11, 21) angeordnet sind.

4. Kieferknochenimplantat nach einem der vorhergehenden Ansprüche, **dadurch gekennzeichnet, dass** im Bereich der Austrittsöffnung bzw. Austrittsöffnungen (18, 19, 28) Hinterschnitte oder vergleichbare Strukturen zum gezielten Zurückhalten eines Teils des durch den Kanal (14, 24) und die Austrittsöffnung bzw. Austrittsöffnungen (18, 19, 28) gepressten Verankerungsmittels vorgesehen sind.

5. Kieferknochenimplantat nach einem der vorhergehenden Ansprüche, **dadurch gekennzeichnet, dass** die Austrittsöffnung bzw. Austrittsöffnungen (29) als eine offen poröse Struktur in dem Haltabschnitt (21) ausgebildet ist bzw. sind.

6. Implantatsystem mit einem Kieferknochenimplantat (10, 20) nach einem der vorhergehenden Ansprüche und einem Verankerungsmittel zum Verankern des Knochenimplantates (10, 20) in einem Knochen (K), wobei das Verankerungsmittel ein mittels eines biokompatiblen, wasserlöslichen Lösungsmittels gelöstes und so verflüssigtes Polymer oder Polymergemisch ist.

7. Implantatsystem nach Anspruch 6, **dadurch gekennzeichnet, dass** das Polymer oder Polymergemisch ein thermoplastischer Kunststoff ist.

8. Implantatsystem nach einem der vorhergehenden Ansprüche 6 oder 7, **dadurch gekennzeichnet, dass** das Polymer oder Polymergemisch ein biologisch abbaubares Polymer oder Polymergemisch ist.

9. Implantatsystem nach Anspruch 8, **dadurch gekennzeichnet, dass** das Polymer oder Polymergemisch in einem lebendigen menschlichen oder tierischen Körper Abbauzeiten von 12 bis 36 Monaten aufweist.

10. Implantatsystem nach einem der vorhergehenden Ansprüche 6 bis 9, **dadurch gekennzeichnet, dass** das Polymer oder Polymergemisch gewählt ist aus einem oder mehreren der folgenden Polymere: Polylactide, Polyglycolide, Polycaprolactone, Polyanhydride, Polyamide, Polyuretane, Polyesteramide, Polyorthoester, Polydioxanone, Polyacetale, Polyketale, Polycarbonate, Polyorthocarbonate, Polyphosphazene, Polyhydroxybutyrate, Polyhydroxyvalerate, Polyalkylenoxalate, Polyalkylensuccinate, Poly(aminosäuren) und Copolymere, Terpolymere oder Kombinationen oder Mischungen dieser Materialien.

11. Implantatsystem nach einem der vorhergehenden Ansprüche 6 bis 10, **dadurch gekennzeichnet, dass** das biokompatible, wasserlösliche Lösungsmittel ausgewählt ist aus einem der folgenden Lösungsmittel: N-Methyl-2-Pyrrolidon, 2-Pyrrolidon, Ethanol, Propylenglycol, Methylacetat, Ethylacetat, Methylethylketon, Dimethylsulfoxid, Caprolactam, Oleinsäure und 1-Dodecylazacycloheptan-2-on oder aus einer beliebigen Kombination oder Mischung dieser Stoffe.

12. Implantatsystem nach einem der vorhergehenden Ansprüche 6 bis 11, **dadurch gekennzeichnet, dass** das Verankerungsmittel neben dem wasserlöslichen biokompatiblen Lösungsmittel und dem Polymer oder Polymergemisch ferner ein Knochenersatzmaterial enthält, insbesondere Tricalciumphosphat und/oder Hydroxiapatit.

13. Implantatsystem nach Anspruch 12, **dadurch gekennzeichnet, dass** das Knochenersatzmaterial in Form kleiner poröser Teilchen, insbesondere Kügelchen, beigemengt ist mit Größen zwischen 0,02 und 0,7 mm.

## Claims

1. A jawbone implant for insertion into a human or animal jawbone (K) of a living body for permanent positioning and for use with an anchoring means made of a polymer or polymer mixture which is dissolved by means a biocompatible, water-soluble solvent, and which is hence liquefied, for anchoring the jawbone implant in a bone, whereas the jawbone implant includes a holding section (11, 21) to be inserted into the bone (K) and a functional section (13, 23) connected to said holding section (11, 21), as well as at least a channel (14, 24) accessible from an access opening (15, 25) outside the holding section (11, 21) and extending inside the jawbone (10, 20), which emerges in a region of the holding section (11, 21) in at least one outlet opening (18, 19, 28), **characterised in that** the channel (14) or a branch channel (16, 17, 26) connected to the channel (14) runs tilted in the direction of a distal end (12, 22) of the holding section (11, 21) in the region of said at least one outlet opening (18, 19, 28).

2. A jawbone implant according to claim 1, **characterised in that** it consists at least in the holding section (11, 21) of a biocompatible metal, of a biocompatible ceramic or of a biocompatible synthetic material or any combination of said materials.

3. A jawbone implant according to any of the preceding claims, **characterised in that** it includes several outlet openings (18, 19, 28) which are arranged on different positions of the circumference of the holding section (11, 21).

4. A jawbone implant according to any of the preceding claims, **characterised in that** undercuts or comparable structures are provided in the region of the outlet opening or outlet openings (18, 19, 28), for targeted retention of a portion of the anchoring means compressed through the channel (14, 24) and the outlet opening or outlet openings (18, 19, 28).

5. A jawbone implant according to any of the preceding claims, **characterised in that** the outlet opening or outlet openings (29) is or are designed as an open porous structure in the holding section (21).

6. An implant system having a jawbone implant (10, 20) according to any of the preceding claims and an anchoring means for anchoring the jawbone implant (10, 20) in a bone (K), whereas the anchoring means is a polymer or polymer mixture dissolved by means of a biocompatible, water-soluble solvent and hence liquefied.

7. An implant system of claim 6, **characterised in that** the polymer or polymer mixture is a thermoplastic synthetic material.

8. An implant system according to any of the preceding claims 6 or 7, **characterised in that** the polymer or the polymer mixture is a biodegradable polymer or polymer mixture.

9. An implant system of claim 8, **characterised in that** the polymer or polymer mixture in a living human or animal body includes decomposition times of 12 to 36 months.

10. An implant system according to any of the preceding claims 6 to 9, **characterised in that** the polymer or polymer mixture is selected from one or several of the following polymers: polyactids, polyglycolids, polycaprolactone, polyanhydrides, polyamides, polyurethane, polyesteramides, polyorthoester, polydioxanone, polyacetals, polyketals, polycarbonates, polyorthocarbonates, polyphosphazenes, polyhydroxybutyrates, polyhydroxybutyrates, polyalkylenoxalates, polyalkylensuccinates, poly(amino acids) and copolymers, terpolymers or combinations or mixtures of said materials.

11. An implant system according to any of the preceding claims 6 to 10, **characterised in that** the biocompatible, water-soluble solvent is selected from one of the following solvents: N-methyl-2-pyrrolidone, 2-pyrrolidone, ethanol, propylene glycol, methyl acetate, ethyl acetate, methyl ethyl ketone, dimethyl sulfoxide, caprolactam, oleic acid and 1-Dodecylazacycloheptan-2-one or from any combination or mixture of said matters.

12. An implant system according to any of the preceding claims 6 to 11, **characterised in that** the anchoring means, in addition to the water-soluble biocompatible solvent and the polymer or polymer mixture also contains a bone substitute material, in particular tricalcium phosphate and/or hydroxiapatite.

13. An implant system of claim 12, **characterised in that** the bone substitute material in the form of small porous particles, in particular spherules, is mixed at sizes comprised between 0.02 and 0.7 mm.

## Revendications

1. Implant pour mâchoire pour insertion dans une mâchoire humaine ou animale (K) d'un organisme vivant pour positionnement permanent et pour utilisation avec un moyen d'ancrage composé d'un polymère ou mélange polymère qui est dissout à l'aide d'un solvant biocompatible soluble dans l'eau et qui est donc liquéfié, pour ancrer l'implant pour mâchoire dans un os, où l'implant pour mâchoire comprend une partie de retenue (11, 21) à introduire dans l'os (K) et une partie fonctionnelle (13, 23) connectée à ladite partie de retenue (11, 21), de même qu'au moins un canal (14, 24) accessible à partir d'une ouverture d'accès (15, 25) en dehors de la partie de retenue (11, 21) et s'étendant à l'intérieur de la mâchoire (10, 20), qui émerge dans une zone de la partie de retenue (11, 21) dans au moins une ouverture de sortie (18, 19, 28), **caractérisé en ce que** le canal (14) ou un canal de dérivation (16, 17, 26) connecté au canal (14) est incliné dans la direction d'une extrémité distale (12, 22) de la partie de retenue (11, 21) dans une zone de ladite au moins une ouverture de sortie (18, 19, 28).

2. Implant pour mâchoire selon la revendication 1, **caractérisé en ce qu'**il se compose au moins dans la partie de retenue (11, 21) d'un métal biocompatible, d'une céramique biocompatible ou d'un matériau synthétique biocompatible ou toute combinaison desdits matériaux.

3. Implant pour mâchoire selon l'une quelconque des revendications précédentes, **caractérisé en ce qu'**il comprend plusieurs ouvertures de sortie (18, 19, 28), qui sont agencées en différentes positions de la circonférence de la partie de retenue (11, 21).

4. Implant pour mâchoire selon l'une quelconque des revendications précédentes, **caractérisé en ce que** des contre-dépouilles ou structures comparables sont prévues dans la zone de l'ouverture de sortie ou des ouvertures de sortie (18, 19, 28), pour le maintien ciblé d'une partie du moyens d'ancrage comprimé à travers le canal (14, 24) et l'ouverture de sortie ou les ouvertures de sortie (18, 19, 28).

5. Implant pour mâchoire selon l'une quelconque des revendications précédentes, **caractérisé en ce que** l'ouverture de sortie ou les ouvertures de sortie (29) est ou sont conçues comme structure poreuse ouverte dans la partie de retenue (21).

6. Système d'implant présentent un implant pour mâchoire (10, 20) selon l'une quelconque des revendications précédentes, et un moyen d'ancrage pour ancrer l'implant pour mâchoire (10, 20) dans un os (K), où le moyen d'ancrage est un polymère ou un mélange polymère dissout à l'aide d'un solvant biocompatible soluble dans l'eau et donc liquéfié.

7. Implant conforme à la revendication 6, **caractérisé en ce que** le polymère ou le mélange polymère est un matériau synthétique thermoplastique.

8. Système d'implant selon l'une quelconque des revendications 6 ou 7, **caractérisé en ce que** le polymère ou le mélange polymère est un polymère ou un mélange polymère biodégradable.

9. Système d'implant conforme à la revendication 8, **caractérisé en ce que** le polymère ou le mélange polymère chez un corps vivant humain ou animal présente des temps de décomposition de 12 à 36 mois.

10. Système d'implant selon l'une quelconque des revendications 6 à 9, **caractérisé en ce que** le polymère ou le mélange polymère est sélectionné parmi un ou plusieurs des polymères suivants: polyactides, polyglycolides, polycaprolactones, polyanhydridures, polyamides, polyuréthanes, polyesteramides, polyorthoester, polydioxanone, polyacétaux, polykétals, polycarbonates, polyorthocarbonates, polyphosphazènes, polyhydroxybutyrates, polyhydroxybutyrates, polyalkylènoxalates, polyalkylène succinates, poly(amino acides) et copolymères, terpolymères ou combinaisons ou mélanges desdits matériaux.

11. Système d'implant selon l'une quelconque des revendications 6 à 10, **caractérisé en ce que** le solvant biocompatible, soluble dans l'eau est sélectionné parmi l'un des solvants suivants: N-méthyle-2-pyrrolidone, 2-pyrrolidone, éthanol, propylène glycol, méthyle acétate, éthyle acétate, méthyle éthyle cétone, diméthyle sulfoxide, caprolactam, acide oléïque et 1-dodécylazacycloheptan-2-one ou toute combinaison ou mélange desdites matériaux.

12. Système d'implant selon l'une quelconque des revendications 6 à 11, **caractérisé en ce que** le moyen d'ancrage, outre le solvant biocompatible soluble dans l'eau et le polymère ou mélange polymère contient également un matériau de substitution osseux, en particulier du tricalcium phosphate et/ou de l'hydroxipatite.

13. Système d'implant conforme à la revendication 12, **caractérisé en ce que** le matériau de substitution osseux sous forme de petites particules poreuses, en particulier des sphérules, est mélangé à des tailles comprises entre 0,02 et 0,7 mm.
